# EUROPEAN PATENT APPLICATION

(11) **EP 4 349 857 A1**
(43) Date of publication of application: **10.04.2024**
(21) Application number: 22811624.0
(22) Date of filing: 24.05.2022
(51) Int. Cl.: C07K 14/705, C07K 16/28, C07K 14/725, C12N 5/0783, A61K 35/17, C12N 15/62, A61P 35/00

(54) **MONOBODY-BASED CHIMERIC ANTIGEN RECEPTOR AND IMMUNE CELL INCLUDING SAME**

(30) Priority: 25.05.2021 KR 20210067276
(71) Applicant: Vaxcell-Bio, Jeollanam-do 58141 (KR)
(72) Inventor: RHEE, Joon Haeng, Gwangju 61903 (KR)
(74) Representative: Marks & Clerk LLP
(86) International application number: PCT/KR2022/007379
(87) International publication number: WO 2022/250431

(57) **Abstract**

The present invention relates to a monobody-based chimeric antigen receptor. The monobody-based chimeric antigen receptor possesses an extracellular ligand binding domain comprising a monobody. In addition, the present invention relates to an immune cell having the monobody-based chimeric antigen receptor expressed on the cell surface membrane thereof. Such monobody-based CAR-immune cells 1) can target a receptor expressed on the surface of cancer cells to exhibit an excellent prophylactic or therapeutic effect on cancer, 2) when administered into humans, exhibit low immunogenicity because they use a human FN3-based monobody as an extracellular ligand binding domain, as opposed to animal-derived scFv-based CAR-immune cells, 3) are of high infiltration into tissues due to their smaller sizes than those of scFV, 4) can minimize the side effects that conventional antibody cancer therapy possesses (e.g., development of an autoimmune disease due to non-specific binding), and 5) can be prepared into various types from a pre-constructed monobody library to various cancer cell antigens. Thus, the monobody-based CAR-immune cells can be advantageously used for preventing or treating incurable solid cancer having various antigens.

## Description

### Technical Field

The present invention relates to a monobody-based chimeric antigen receptor. The monobody-based chimeric antigen receptor comprises an extracellular ligand binding domain comprising a monobody. In addition, the present invention relates to an immune cell having the monobody-based chimeric antigen receptor expressed on the cell surface membrane thereof.

### Background Art

When treating solid cancer, it is difficult to completely remove cancer stem cells, remaining cancer cells, or metastatic cancer cells through surgical resection alone, so a combination treatment method that combines surgical resection with radiation therapy, chemotherapy, target therapy, and immunotherapy is being used. In particular, immunotherapy uses the body's immune system, so there are almost no side effects seen with radiation therapy or chemotherapy.

T cells are immune cells that mediate adaptive immunity and account for 75% of all lymphocytes. T cells mostly exist in an inactive state in the blood, but when stimulated by antigens, they change into an active form and can eliminate cells with specific antigens. The action of T cells can be activated by receptors that can recognize antigens (T cell receptor; TCR), co-stimulatory molecules, and cytokines.

However, the TCR of T cells binds to a MHC (major histocompatibility complex) molecule to which a tumor-related antigen is bound, and cancer cells inhibit the expression of the MHC or inhibit the function of T cells by changing the function of immune checkpoints such as CTLA-4 and PD-1 expressed on the surface of T cells. Accordingly, T cell (Chimeric Antigen Receptor T cells; CAR-T), which expresses within the cell a chimeric antigen receptor that can directly recognize tumor-related antigens rather than recognize MHC molecules, or T cell that expresses within the cell an antibody sequence specific for immune checkpoint receptors on cancer cells began to be developed.

The structure of the currently developed chimeric antigen receptor is divided into an scFv (single chain variable fragment) portion that recognizes the antigen, a transmembrane domain, and a signaling domain that transmits signals inside a cell. Pharmaceutical companies such as Novartis, Gilead, Celgene, and Abclon, as well as clinicians, have developed CAR-T using scFv that specifically binds to the CD19 antigen of blood cancer cells as a treatment for blood cancer (International Publication Nos. WO201616473, WO2015157252, WO2018023025, WO2017211900, WO2017211900, and WO2016028896).

CAR-based immune cell therapy combining various genetic engineering technologies is absolutely required for solid cancer. However, in the case of solid cancer, there is no progress due to the heterogeneity of expressing different antigens for each patient, the complex nature of the tumor microenvironment such as a hypoxic environment (hypoxia), and restrictions on the movement and activity of T cells.

Ephrin receptor is a membrane receptor tyrosine kinase (RTK) of 108 kDa in size, and is divided into three parts: on the outside of the cell membrane, it has a ligand binding domain, a cysteine-rich region, and two fibronectin III repeat structures; in the middle, it has a transmembrane part; and on the cytoplasmic side, it has a kinase activity domain (Labrador et al., 1997; Pasquale, 1997). The ephrin receptor is a protein that binds to the ligand ephrin and transmits external signals into the cell through the phosphorylation process, and is responsible for cell proliferation, cell migration, differentiation, synaptic transmission of nerve cells, tissue remodeling, osteocyte differentiation, and angiogenesis (Pasquale EB. Cell. 133:38-52, 2008; Barquilla A et al., Annu. Rev. Pharmacol. Toxicol. 55:465-487, 2015). Depending on the structure of the ephrin receptor and the ligand-receptor specificity, the ephrin receptor is divided into 9 types of group A (EphA1-8 and EphA10) and 5 types of group B (EphB1-4 and EphB6) (Ieguchi K. Endocr Metab Immune Disord Drug Targets. 15:119-128, 2015; Eph Nomenclature Committee. Cell. 90:403-404, 1997; Lindberg RA et al., Mol Cell Biol. 10(12):6316-6324, 1990; Davis S et al., Science. 266(5186):816-819, 1994). Among these, ephrin receptor A2 (EphA2) is expressed in very small amounts in normal cells, but is non-specifically overexpressed in most cancer cells, such as liver cancer, prostate cancer, pancreatic cancer, head and neck cancer, stomach cancer, large intestine cancer, lung cancer, cervical cancer, and ovarian cancer, and promotes cancer carcinogenesis, metastasis, angiogenesis, and resistance of tumors. EphA2 has approximately 65% protein homology with EphA1, is found in almost the same location in mouse lung, is overexpressed together with EphA1 and its ligand, Ephrin-A1, in several malignant tumors, including non-small cell lung cancer, and is involved in carcinogenesis and malignization progression through interaction with EphA2/A1-Ephrin-A1 (Naj AC et al., Nat Genet. 43:436-441, 2011; Finney AC et al., Circulation. 136:566-582, 2017).

### Prior Art Document

### Patent Document

(Patent Document 1) KR10-2020-0105840 A
(Patent Document 2) KR10-2018-0121904 A
(Patent Document 3) KR10-2018-0127344 A
(Patent Document 4) KR10-2007-0107721 A
(Patent Document 5) KR10-1528939 B1
(Patent Document 6) KR10-1521668 B1
(Patent Document 7) KR10-1636882 B1
(Patent Document 8) KR10-2018-0134347 A
(Patent Document 9) KR10-2157197 B1

### Non-Patent Document

(Non-Patent Document 1) Park SH, Park S, Kim DY, Pyo A, Kimura RH, Sathirachinda A, Choy HE, Min JJ, Gambhir SS, Hong Y Isolation and Characterization of a Monobody with a Fibronectin Domain III Scaffold That Specifically Binds EphA2. PLoS One. 2015 Jul 15;10(7):e0132976. doi: 10.1371/journal.pone.0132976. eCollection 2015.PMID: 26177208
(Non-Patent Document 2) Kim MA, Yoon HS, Park SH, Kim DY, Pyo A, Kim HS, Min JJ, Hong Y. Engineering of monobody conjugates for human EphA2-specific optical imaging. PLoS One. 2017 Jul 7;12(7):e0180786. doi: 10.1371/journal.pone.0180786. eCollection 2017.PMID: 28686661
(Non-Patent Document 3) Pyo A, You SH, Sik Kim H, Young Kim J, Min JJ, Kim DY, Hong Y. Production of(64)Cu-labeled monobody for imaging of human EphA2-expressing tumors. Bioorg Med Chem Lett. 2020 Jul 15;30(14):127262. doi: 10.1016/j.bmcl.2020.127262. Epub 2020 May 15. PMID: 32527560

### Detailed Description of Invention

### Technical Problem

CAR-immune cells (e.g., CAR-T cells) that used the single chain variable fragment (scFv) of a conventional antibody as an extracellular ligand binding domain had the following problems.
1) Since it is an animal-derived protein obtained from a mouse monoclonal antibody, it has strong binding to cancer antigens, leading to serious side effects such as cytokine storm (cytokine release syndrome, CRS), graft-versus-host disease, and tumor lysis syndrome. Excessive activation of the immune system causes a rapid contraction of the immune system, reducing its long-term tumor inhibitory effect.
2) Unlike blood cancers that use CD19 as a single antigen, solid cancers have a variety of antigens (heterogeneity) that are different for each patient, and animal-based antibody information must be used to construct a diverse library for these cancer antigens, which requires a lot of money, a long period of time, and a lot of effort.
3) Antibodies, double antibodies, and scFvs are large in size. Therefore, when used in the treatment of solid cancer, it is difficult to exhibit sufficient therapeutic effect due to their low penetration into tissues.
4) When a bicistronic scFv, that is, two antigen recognition sites, is expressed in T cells or NK cells, the affinity of the scFv for the antigen is reduced, and therefore, it may exhibit low therapeutic effect or have side effects due to non-specific binding.
5) Even if scFv-based CAR-immune cell therapeutic agent is administered, mutations occur in the cancer cell epitope that is the target of CAR, resulting in a high incidence of antigen evasion.

In order to solve these problems with scFv, a monobody was developed. The monobody is similar to scFv and can recognize antigens, and has the advantage of having high infiltration into the tissue due to its smaller size compared to scFv. However, it has the following problems when used to treat cancer cells.
1) The binding ability to antigen is relatively low compared to antibodies and scFv.
2) Since the monobody itself is not toxic to cancer cells, various manipulations (e.g., combining anticancer agents, fusing T cells or NK cells, etc.) are required to use it as a cancer therapeutic agent.
3) The monobody itself has low stability in serum, and for example, in order to use the monobody as a contrast agent, a relatively large amount must be administered (e.g., exceeding about 60 ug).
4) The monobody has a short half-life in the body (<24h) and must be injected frequently.

In order to solve the above problems, the present inventors have developed a novel monobody-based CAR-immune cell that can selectively recognize and bind to cancer cells, has excellent penetration in vivo to exhibit high effectiveness, is not harmful even when administered in large doses, and can induce immune memory while remaining continuously in the tissue.

In order to solve the problems of the prior art described above, an object of the present invention is to provide a monobody-based chimeric antigen receptor and an immune cell expressing the same on a cell surface membrane.

### Solution to Problem

The present invention relates to a monobody-based chimeric antigen receptor comprising an extracellular ligand binding domain comprising a monobody.

The monobody may specifically bind to a protein selected from the group consisting of CRL1, ephrin receptor, EphA2, β-galactosidase, RAS, Abl kinase, VEGFR2, MBP, SARS-CoV-2, Bcr-Abl kinase, STAT3, EGFR, VEGFR2, SH3 domain of human Lyn tyrosine kinase, MLKL, Fluc homologues, mitogen-activated protein kinase (MAPK), ERK2, MAPK14, kinase SH2 domain, SUMO, AurA, WDR5, Flue family, GFP, receptor binding domain of SARS-CoV-2, SH3 domain of FYN, SH2 domain of ABL, SUMO1, Bcr-Abl, GPR56 ECR, PD-L1, glypican-3, PCSK9, Gp41, CD4, and IL-23.

The monobody may specifically bind to ephrin receptor, EphA2, or human EphA2.

The monobody may comprise the amino acid sequence of SEQ ID NO: 8.

The monobody-based chimeric antigen receptor may further comprise a transmembrane domain; and an intracellular signaling domain.

The transmembrane domain may be at least one selected from the group consisting of T-cell receptor alpha chain, T-cell receptor beta chain, T-cell receptor zeta chain, CD8 alpha chain, CD8 beta chain, CD28, CD3epsilon, CD45, CD4, CD5, CD8, CD9, CD16, CD22, CD33, CD37, CD64, CD80, CD86, CD134, CD137, CD154, a portion thereof, and a combination thereof.

The intracellular signaling domain may include at least one signaling domain selected from the group consisting of TCRzeta, FcRgamma, FcRbeta, FcRepsilon, CD3gamma, CD3delta, CD3epsilon, CD3zeta, CD5, CD22, CD79a, CD79b, and CD66d.

The intracellular signaling domain may include at least one selected from the group consisting of an OX40 domain, a CD2 domain, a CD27 domain, a CD28 domain, a CDS domain, an ICAM-1 domain, a LFA-1 domain, and a 4-1BB domain.

The monobody-based chimeric antigen receptor of the present invention may further comprise a hinge.

The present invention may relate to a polynucleotide comprising a nucleic acid sequence encoding the monobody-based chimeric antigen receptor of the present invention.

The present invention may relate to an expression vector comprising the polynucleotide.

The present invention may relate to an engineered immune cell expressing the monobody-based chimeric antigen receptor on a cell surface membrane.

The immune cell may be a white blood cell, a neutrophil, an eosinophil, a basophil, a monocyte, a lymphocyte, a T cell, a cytotoxic T cell, a natural killer T cell, a dendritic cell, or a combination thereof.

The present invention may relate to a pharmaceutical composition for treating cancer, comprising the immune cell.

### Effects of Invention

The monobody-based chimeric antigen receptor according to the present invention, an immune cell expressing the same on a cell surface membrane, and a composition for preventing or treating cancer using the same can exhibit the following effects.
1) Side effects (e.g. hair loss and digestive disorders) can be reduced, which occur by inhibiting the growth of normal cells in conventional radiation therapy or chemotherapy such as DNA nucleotide analogues (cisplatin series and 5-FU series) that only suppress rapid cell proliferation.
2) Since it uses a small-sized monobody based on human FN3, autoimmune disease side effects that occur in target antibody therapy, which is used in animal-derived antibody-based anticancer therapy, and chimeric T-cell therapy that uses scFvs obtained from a library of mouse monoclonal antibodies, and cancer cell metastasis due to resistance to anticancer agent can be minimized.
3) This overcomes the limitations of monobodies, which were used only for diagnosis due to their lack of cancer-killing ability, instability, and short lifespan, and can effectively inhibit or eliminate solid cancers expressing cancer antigens (e.g., EphA2). Therefore, diagnosis and treatment of cancer can be performed simultaneously.
4) The monobody-based CAR-immune cell can exhibit excellent anticancer effects compared to solid cancer treatment using conventional T-cells alone. For example, it can be used as an immune anticancer agent for treating or preventing solid cancers, such as pancreatic cancer, prostate cancer, and ovarian cancer, which express EphA2 as an antigen.
5) FN3 monobody has the advantage of being able to produce various types of monobodies against target antigens. This makes it possible to prepare a monobody-based CAR-immune cell specific for various antigens, making it very effective in treating cancer.
6) A monobody-based CAR-immune cell is smaller than other CAR-immune cells (e.g., the size of a monobody is about 1/3 of the size of scFv), so it is easier to infiltrate into cancer tissue than scFv-based CARs. In addition, a monobody-based CAR-immune cell can carry up to four monobody sequences that recognize cancer antigens into the vector by considering virus packing when attempting to construct a multivalent CAR into a viral vector, which is an advantage that can overcome the diversity (heterogeneity) of cancer antigens, a characteristic of solid tumors. In addition, since it has sufficient selectivity and specificity for cancer antigens, it can block immune cell evasion by cancer and effectively prevent or treat cancer.

### Brief Description of Drawings

Figure 1 shows the structure of a vector expressing a monobody-based chimeric antigen receptor.
Figures 2a and 2b schematically illustrate a method for producing a vector expressing a monobody-based chimeric antigen receptor.
Figure 3 shows the structure of a monobody-based chimeric antigen receptor expressed on the cell membrane surface.
Figure 4 shows the results obtained by analyzing the expression level of the vector in immune cells into which the vector expressing the E1 monobody chimeric antigen receptor was introduced by FACS.
Figure 5 shows the results obtained by analyzing the activity level of T cell activating factors CD25 and CD69 in VEC-1 and VEC-2 immune cells by FACS.
Figure 6 shows the results obtained by analyzing the expression level of EphA2 protein in solid cancer cells (stomach cancer, large intestine cancer, pancreatic cancer, ovarian cancer, and prostate cancer) by FACS.
Figure 7 shows the results obtained by analyzing the cancer cell killing ability of VEC-1 and VEC-2 immune cells using RTCA.
Figure 8 shows the results obtained by analyzing the cancer cell killing ability of E1 monobody CAR-T (VEC-1) immune cells using 3D co-culture.
Figure 9 shows the changes in tumor size in a xenograft mouse model.
Figure 10 shows the changes in body weight of mice in a xenograft mouse model.
Figure 11 shows a mouse survival curve graph drawn using the Kaplan-Meier formula in a xenograft mouse model.
Figure 12 is a photograph comparing mouse tumor sizes on the 44th days after tumor transplantation in a xenograft mouse model.
Figure 13 shows IVIS imaging results of comparing the degree of metastasis and proliferation of pancreatic cancer tumors in a pancreatic cancer xenograft mouse model.
Figure 14 shows a pancreatic cancer tumor growth curve in a pancreatic cancer xenograft model analyzed by bioluminescence imaging.

### Best Mode for Carrying out the Invention

Unless specifically defined in the present invention, all technical and scientific terms used in the present invention may have the same meaning as commonly understood by those of ordinary skill in the art in the fields of gene therapy, biochemistry, genetics, and molecular biology.

Practicing the present invention, unless otherwise specified, may utilize conventional techniques in cell biology, cell culture, molecular biology, microbiology, recombinant DNA, and immunology, which may be within the skill of the art.

### Monobody-based chimeric antigen receptor

The present invention relates to a monobody-based chimeric antigen receptor (CAR).

The monobody-based chimeric antigen receptor of the present invention may comprise an extracellular ligand binding domain.

As used herein, the term "extracellular ligand binding domain" may refer to an oligopeptide or polypeptide capable of binding a ligand. Preferably, the domain can interact with cell surface molecules. For example, an extracellular ligand binding domain can be selected to recognize a ligand that acts as a cell surface marker on target cells related to any disease.

As used herein, the term "monobody" may specifically bind to a ligand or antigen present on the surface of a target cell. The monobody can be selected in various ways depending on the desired target. For example, the monobody may specifically bind to a protein selected from the group consisting of CRL1, ephrin receptor, EphA2, β-galactosidase, RAS, Abl kinase, VEGFR2, MBP, SARS-CoV-2, Bcr-Abl kinase, STAT3, EGFR, VEGFR2, SH3 domain of human Lyn tyrosine kinase, MLKL, Fluc homologues, mitogen-activated protein kinase (MAPK), ERK2, MAPK14, kinase SH2 domain, SUMO, AurA, WDR5, Flue family, GFP, receptor binding domain of SARS-CoV-2, SH3 domain of FYN, SH2 domain of ABL, SUMO1, Bcr-Abl, GPR56 ECR, PD-L1, glypican-3, PCSK9, Gp41, CD4, and IL-23, but is not limited thereto.

In addition, the monobody may be known.

The monobody may specifically bind to ephrin receptor, EphA2, or human EphA2.

The monobody may have at least 70%, preferably at least 80%, more preferably at least 90%, 95%, 97% or 99% sequence identity to the amino acid sequence of SEQ ID NO: 8.

The monobody-based CAR may further comprise at least one selected from the group consisting of a hinge, a transmembrane domain, and an intracellular signaling domain.

In addition, the monobody-based CAR is expressed on the surface membrane of cells. Accordingly, the monobody-based CAR may comprise a transmembrane domain. The distinguishing characteristic of an appropriate transmembrane domain is that it is expressed on the surface of a cell, preferably an immune cell in the present invention (particularly, a lymphocyte cell or a NK cell), and includes its ability to interact together to induce a cellular response of the immune cell against a predetermined target cell. The transmembrane domain may be derived from natural or synthetic sources. The transmembrane domain may be derived from any membrane-bound or transmembrane protein.

The transmembrane domain may be at least one selected from the group consisting of T-cell receptor alpha chain, T-cell receptor beta chain, T-cell receptor zeta chain, CD8 alpha chain, CD8 beta chain, CD28, CD3epsilon, CD45, CD4, CD5, CD8, CD9, CD16, CD22, CD33, CD37, CD64, CD80, CD86, CD134, CD137, CD154, a portion thereof, and a combination thereof.

The hinge and transmembrane domain may comprise human CD8 alpha chain or a portion thereof. The hinge and transmembrane domain may comprise the amino acid sequence of SEQ ID NO: 9, and preferably may comprise an amino acid sequence having at least 70%, preferably at least 80%, more preferably at least 90%, 95%, 97% or 99% sequence identity to the amino acid sequence of SEQ ID NO: 9.

The intracellular signaling domain of a monobody-based CAR may be responsible for intracellular signaling after binding the extracellular ligand binding domain to the target, resulting in activation of immune cells and immune responses. The signaling domain may be responsible for activation of at least one of the normal effector functions of the immune cell expressing the monobody-based CAR. For example, the effector function of a T cell may be a cytotoxic activity or a helper activity, including secretion of cytokines.

As used herein, the term "signaling domain" may refer to a portion of a protein that converts an effector function signal within an immune cell and induces the cell to perform a specific function.

The intracellular signaling domain may comprise at least one selected from the group consisting ofTCRzeta, FcRgamma, FcRbeta, FcRepsilon, CD3gamma, CD3delta, CD3epsilon, CD3zeta, CD5, CD22, CD79a, CD79b, and CD66d.

The intracellular signaling domain may include at least one selected from the group consisting of an OX40 domain, a CD2 domain, a CD27 domain, a CD28 domain, a CDS domain, an ICAM-1 domain, a LFA-1 domain, and a 4-1BB domain.

The intracellular signaling domain may include at least one selected from the group consisting of a CD28 domain, a 4-1BB domain, and a CD3 zeta domain, and preferably may comprise a CD28 domain and a CD3 zeta domain, or a 4-1BB domain and a CD3 zeta domain.

The CD28 domain may comprise the amino acid sequence of SEQ ID NO: 10 or an amino acid sequence having at least 70%, preferably at least 80%, more preferably at least 90%, 95%, 97% or 99% sequence identity to the amino acid sequence of SEQ ID NO: 10.

The 4-1BB domain may comprise the amino acid sequence of SEQ ID NO: 11 or an amino acid sequence having at least 70%, preferably at least 80%, more preferably at least 90%, 95%, 97% or 99% sequence identity to the amino acid sequence of SEQ ID NO: 11.

The CD3 zeta domain may comprise the amino acid sequence of SEQ ID NO: 12 or an amino acid sequence having at least 70%, preferably at least 80%, more preferably at least 90%, 95%, 97% or 99% sequence identity to the amino acid sequence of SEQ ID NO: 12.

### Polynucleotide and vector

The present invention may relate to a polynucleotide comprising a nucleic acid sequence encoding a monobody-based CAR, or to a vector comprising the nucleic acid sequence or polynucleotide. Here, the vector may refer to an expression vector.

The polynucleotide may comprise the nucleic acid sequence of SEQ ID NO: 2. The polynucleotide may comprise a nucleic acid sequence having at least 70%, preferably at least 80%, more preferably at least 90%, 95%, 97% or 99% sequence identity to the nucleic acid sequence of SEQ ID NO: 2.

The polynucleotide may comprise one or more nucleic acid sequences of SEQ ID NOs: 1 to 6, or may comprise one or more of each nucleic acid sequence having at least 70%, preferably at least 80%, more preferably at least 90%, 95%, 97% or 99% sequence identity to the nucleic acid sequences of SEQ ID NOs: 1 to 6.

A nucleic acid sequence encoding a monobody-based CAR may be codon-optimized for expression in human cells.

As used herein, the term "vector" may refer to a construct capable of delivering one or more gene(s) or sequence(s) of interest into a host cell, preferably capable of expressing them. For example, the vector includes viral vectors, naked DNA or RNA expression vectors, plasmids, cosmids or phage vectors, DNA or RNA expression vectors associated with cationic condensing agents, DNA or RNA expression vectors encapsulated in liposomes, and certain eukaryotic cells, such as producer cells, but is not limited thereto.

Viral vectors may include retroviruses, adenoviruses, parvoviruses (e.g., adeno-associated viruses), coronaviruses, negative strand RNA viruses, such as ortho-myxoviruses (e.g., influenza viruses), rhabdoviruses (e.g., rabies and vesicular stomatitis viruses), paramyxoviruses (e.g., measles and Sendai), positive strand RNA viruses, such as picornavirus and alphavirus, and double-stranded DNA viruses, including adenoviruses, herpesviruses (e.g., herpes simplex virus types 1 and 2, Epstein-Barr virus, cytomegalovirus), and poxviruses (e.g., vaccinia, fowl pox, and canary pox). Other viruses that can be used as vectors may be, for example, Norwalk virus, togavirus, flavivirus, reovirus, papovavirus, hepadnavirus, and hepatitis virus. Examples of retroviruses may include avian leukosis-sarcoma, mammalian C-type, B-type viruses, D-type viruses, HTLV-BLV group, lentiviruses, and spumaviruses.

### Method of manipulating immune cells

The present invention may relate to a method of producing an immune cell for immunotherapy, comprising introducing a monobody-based CAR into an immune cell; and expanding the cell.

The present invention may relate to a method of manipulating an immune cell, comprising providing an immune cell; and introducing a polynucleotide into the immune cell.

The present invention may relate to a method of manipulating an immune cell, comprising providing an immune cell; and expressing a monobody-based CAR on the surface of the cell.

The present invention may comprise modifying an immune cell with at least one polynucleotide encoding a monobody-based CAR; and expressing the polynucleotide into the cell. A method of producing or manipulating the immune cell of the present invention may be an in vitro or ex vivo method. The polynucleotide may be included in a lentiviral vector for stable expression in cells.

### Engineered immune cell

The present invention may relate to an isolated cell or cell line that can be obtained by the above method of producing or manipulating an immune cell. Here, the isolated cell may comprise a monobody-based CAR.

The isolated cell of the present invention may comprise a population of CARs comprising different extracellular ligand binding domains. In particular, the isolated cell may comprise an exogenous polynucleotide sequence encoding a CAR.

The immune cell of the present invention may refer to a cell of hematopoietic origin that is functionally involved in the initiation and/or execution of innate and/or adaptive immune responses. The immune cell of the present invention may be derived from stem cells. The stem cells may be adult stem cells, non-human embryonic stem cells, non-human stem cells, umbilical cord blood stem cells, progenitor cells, bone marrow stem cells, induced multipotent stem cells, pluripotent stem cells, or hematopoietic stem cells.

The immune cell of the present invention may be a human CD3+ T cell. In one embodiment, the isolated cell may be a dendritic cell, a killer dendritic cell, a mast cell, a NK-cell, a B-cell, or a T-cell selected from the group consisting of a inflammatory T-lymphocyte, a cytotoxic T-lymphocyte, a regulatory T-lymphocyte, or a helper T-lymphocyte. In one embodiment, the cell may be derived from the group consisting of a CD4+ T-lymphocyte and a CD8+ T-lymphocyte.

The cells can be obtained from a subject through a variety of non-limiting methods. The cells can be obtained from a number of non-limiting sources, including peripheral blood mononuclear cells, bone marrow, lymph node tissue, umbilical cord blood, thymic tissue, tissue from the site of infection, ascites, pleural effusion, spleen tissue, and tumor.

Any T cell line available and known to those of ordinary skill in the art can be used. The cells may be derived from a healthy donor, from a patient diagnosed with cancer, or from a patient diagnosed with an infection. The cells may be a portion of a mixed population of cells exhibiting different phenotypic characteristics. A cell line obtained from T cells engineered according to the above-described method can be used, and the immune cell according to the present invention may be resistant to immunosuppressive treatment.

The immune cell may be a white blood cell, a neutrophil, an eosinophil, a basophil, a monocyte, a lymphocyte, a T cell, a cytotoxic T cell, a natural killer T cell, a dendritic cell, or a combination thereof, but is not limited thereto.

### Treatment using monobody-based CAR-immune cell

A monobody-based CAR-immune cell, an engineered immune cell, or a population of these cells can be used as a pharmaceutical composition for preventing or treating cancer.

The present invention may relate to a pharmaceutical composition for preventing or treating cancer, comprising a monobody-based CAR-immune cell or an engineered immune cell.

The present invention may relate to a method for preventing or treating cancer in a subject in need thereof, comprising administering a monobody-based CAR-immune cell to the subject in need thereof.

The present invention may relate to a use of a monobody-based CAR-immune cell for the manufacture of a medicament for preventing or treating cancer.

The present invention may relate to a monobody-based CAR-immune cell for use in the prevention or treatment of cancer.

As used herein, the term "subject" is a mammal, more preferably a human. Mammals include, but are not limited to, livestock, pets, primates, horses, dogs, cats, mice, and rats.

In the present invention, the treatment may be a part of autoimmune therapy or a part of allogeneic immunotherapy. Autologous can mean that the cells, cell line, or population of cells used to treat a subject are obtained from the subject or from a donor compatible for a human leukocyte antigen (HLA). Allogeneic can mean that the cells or population of cells used to treat a subject are not obtained from the subject but from a donor.

The cancer may be melanoma, squamous cell carcinoma, breast cancer, head and neck cancer, thyroid cancer, soft tissue sarcoma, osteosarcoma, testicular cancer, prostate cancer, ovarian cancer, bladder cancer, skin cancer, brain cancer, angiosarcoma, mast cell tumor, leukemia, lymphoma, liver cancer, lung cancer, pancreatic cancer, stomach cancer, kidney cancer, large intestine cancer, hematopoietic tumor, or a metastatic cancer thereof, but is not limited thereto. In addition, the cancer may be a non-solid tumor or a solid cancer.

The non-solid tumor or non-solid cancer may be myeloma, lymphoma, or leukemia, but is not limited thereto.

The solid cancer may be at least one selected from the group consisting of pancreatic cancer, prostate cancer, ovarian cancer, breast cancer, cervical cancer, skin cancer, colorectal cancer, kidney cancer, liver cancer, brain cancer, and lung cancer, but is not limited thereto.

The following examples are provided for illustrative purposes only and do not limit the scope of the invention in any way. Indeed, various modifications of the present invention in addition to those shown and described herein will become apparent to those of ordinary skill in the art from the foregoing description and fall within the scope of the appended claims.

Conventional anticancer immunotherapy is known as a treatment for blood cancer in which cancer cells are eliminated by secretion of various cytokines or attack of surrounding immune cells after recognizing and binding to human cancer antigens using mouse-based antibody molecules, specifically antibody fragments (scFv). However, it has problems such as side effects caused by animal-based antibodies, low infiltration into tissues due to the size of scFv, and the need to recognize at least two or more antigens simultaneously due to the characteristic in which solid cancers express different antigens for each patient (heterogeneity).

In the present invention, in order to compensate for these shortcomings of scFv, it was confirmed that when a monobody based on human fibronectin FN3 is used as an extracellular ligand binding site of a chimeric antigen receptor, it has excellent effect of preventing and treating solid cancer.

### [Example 1]

### Production of vector expressing monobody-based chimeric antigen receptor

### 1. Preparation for production of recombinant lentiviral vector

The following strains and vectors were prepared for the production of a recombinant plasmid vector expressing a monobody-based chimeric antigen receptor.

### (1) Vector to be replicated

The pETh-E1GSE1 vector, which comprises a nucleic acid sequence (SEQ ID NO: 2) expressing a monobody that specifically binds to human EphA2 (hereinafter referred to as E1 monobody), was purchased from research team of Professor Young-jin Hong at the Chonnam National University R&BD Foundation, and a vector expressing a chimeric antigen receptor was prepared. Specifically, the expression vector used was pLenti7.3/V5-DEST^{™} Gateway^{™} Vector from Invitrogen, and it was designed to comprise a nucleic acid sequence expressing a chimeric antigen receptor consisting of CD8α leader sequence-MSLN scFv-CD8 H&TM-CD28-CD3ζ or CD8α leader sequence-MSLN scFv-CD8 H&TM-4-1BB-CD3ζ between 5' UTR and 3' UTR of the pLenti7.3/V5-DEST vector. Two lentiviral vectors were prepared in this way (pLenti7.3::CD8α leader sequence-MSLN scFv-CD8 H&T-CD28-CD3ζ vector and pLenti7.3:: CD8α leader sequence-MSLN scFv-CD8 H&T-4-1BB-CD3ζ vector).

The nucleic acid sequences constituting the monobody-based chimeric antigen receptor in the recombinant plasmid vector are shown in Table 1 below.

**[Table 1]**

| SEQ ID NO | Sequence name | Sequence | Description |
|---|---|---|---|
| SEQ ID NO: 1 | CD8α signal sequence nucleotide | ATGGCCCTGCCTGTGACAG CTTTGCTGCTGCCTCTGGC TCTGCTGCTGCATGCTGCT AGACCT | codon-optimized nucleotide sequence of the amino acid sequence (1-21, NCBI accession # NP_741969.1) of the signal sequence domain of human CD8α |
| SEQ ID NO: 2 | E1 monomer nucleotide | GTTTCTGATGTTCCGAGGG ACCTGGAAGTTGTTGCTGC GACCCCCACCAGCCTACTG ATCAGCTGGTATTATCCGTT TTGTGCGTTTTATTACAGG ATCACTTACGGAGAAACA GGAGGAAATAGCCCTGTCC AGGAGTTCACTGTGCCTCG TCCGTCTGACACTGCTACC ATCAGCGGCCTTAAACCTG GAGTTGATTATACCATCAC TGTGTATGCTGTCACGTGT CTGGGCTCGTATTCTCGCC CAATTTCCATTAATTACCGA ACA | - |
| SEQ ID NO: 3 | CD8α H&TM nucleotide | ACCACCACACCAGCTCCA CGGCCTCCAACACCAGCA CCTACAATTGCCTCTCAGC CCCTGTCTCTGAGGCCAGA AGCTTGTAGACCTGCTGCA | codon-optimized nucleotide sequence of the amino acid sequence (138-206, NCBI accession # |
| | | GGCGGAGCCGTGCATACC AGAGGACTGGATTTCGCCT GCGACATCTACATTTGGGC CCCTCTGGCTGGAACATGT GGCGTCCTGCTGCTGTCTC TCGTGATCACCCTGTACTG C | NP _ 001139345.1) of the hinge and transmembrane protein domain of human CD8α |
| SEQ ID NO: 4 | CD28 nucleotide | AGAAGCAAGAGAAGCCGG CTGCTGCACAGCGACTACA TGAACATGACCCCTAGACG GCCCGGACCTACCAGAAA GCACTACCAGCCTTACGCT CCTCCTCGGGACTTTGCCG CCTATAGATCC | optimized nucleotide sequence of the amino acid sequence (83-123, NCBI accession # NP _001230006.1) of the intracellular signaling domain derived from CD28 |
| SEQ ID NO: 5 | 4-1BB nucleotide | AAGCGGGGCAGAAAGAAG CTGCTGTACATCTTCAAGC AGCCCTTCATGCGGCCCGT GCAGACCACACAAGAGGA AGATGGCTGCTCCTGCAGA TTCCCCGAGGAAGAAGAA GGCGGCTGCGAGCTG | optimized nucleotide sequence of the amino acid sequence (214-255, NCBI accession # NP_001552.2) of the intracellular signaling domain derived from 4-1BB |
| SEQ ID NO: 6 | CD3ζ nucleotide | AGAGTGAAGTTCTCTAGAT CTGCCGACGCTCCTGCCTA CCAGCAGGGACAGAATCA GCTGTACAACGAGCTGAAT CTGGGGCGCAGAGAAGAG TACGACGTGCTGGATAAGA GAAGAGGCAGAGATCCCG AGATGGGCGGCAAGCCCC AGAGAAGAAAGAATCCCC AAGAGGGCCTGTATAATGA GCTGCAGAAAGACAAGAT GGCCGAGGCCTACAGCGA GATCGGAATGAAGGGCGA ACGCAGAAGAGGAAAGGG | optimized nucleotide sequence of the amino acid sequence (60-172, NCBI accession # XP_011508446.1) of the intracellular signaling domain derived from CD3ζ |
| | | CCACGACGGACTGTATCAG GGCCTGAGCACAGCCACC AAGGATACCTATGATGCCC TGCACATGCAGGCCCTGCC ACCTAGG | |

The nucleic acid sequences in Table 1 are expressed in immune cells so that a monobody-based chimeric antigen receptor consisting of the amino acid sequence in Table 2 below can be expressed on the cell surface membrane.

**[Table 2]**

| SEQ ID NO | Sequence name | Sequence | Description |
|---|---|---|---|
| SEQ ID NO: 7 | CD8α signal sequence amino acid | MALPVTALLLPLALLLHAA RP | amino acid sequence corresponding to SEQ ID NO: 1 (1-21 amino acid sequence, NCBI accession # NP_741969.1) |
| SEQ ID NO: 8 | E1 monomer amino acid | VSDVPRDLEVVAATPTSLLI SWYYPFCAFYYRITYGETG GNSPVQEFTVPRPSDTATISG LKPGVDYTITVYAVTCLGSY SRPISINYRT | amino acid sequence corresponding to SEQ ID NO: 2 |
| SEQ ID NO: 9 | CD8α H&TM amino acid | TTTPAPRPPTPAPTIASQPLS LRPEACRPAAGGAVHTRGL DFACDIYIWAPLAGTCGVLL LSLVITLYC | amino acid sequence corresponding to SEQ D NO: 3 (138-206, NCBI accession # NP_001139345.1) |
| SEQ ID NO: 10 | CD28 amino acid | RSKRSRLLHSDYMNMTPRR PGPTRKHYQPYAPPRDFAA YRS | amino acid sequence corresponding to SEQ D NO: 4 (83-123, NCBI accession # LP_00123 0006.1) |
| SEQ ID NO: 11 | 4-1BB amino acid | KRGRKKLLYIFKQPFMRPV QTTQEEDGCSCRFPEEEEGG CEL | amino acid sequence corresponding to SEQ D NO: 5 (214-255, NCBI accession # NP_001552.2) |
| SEQ ID NO: 12 | CD3ζ amino acid | RVKFSRSADAPAYQQGQNQ LYNELNLGRREEYDVLDKR RGRDPEMGGKPQRRKNPQ EGLYNELQKDKMAEAYSEI GMKGERRRGKGHDGLYQG LSTATKDTYDALHMQALPP R | amino acid sequence corresponding to SEQ ID NO: 6 (60-172, NCBI accession # XP_011508446.1) |

### (2) Preparation of cells for replication of vector

Host cell for pETh-E1GSE1 vector: *E. coli* DH5α (F- *end*A1 *glnV44 thi-1 recA1 relA1 gyrA96 deoR nupG purE20* ϕ80d *lacZΔ*M15 Δ(l *acZYA-argF*)U169*,* hsdR17 ( *rK-mK*+)*,* λ-)

Host cell for pLentiviral vector: *E. coli* Stbl3 (F- *mcrB mrrhsd*S20(rB-*,* mB-) *recA*13 *sup*E44 *ara-*14 *galK2 lac*Y1 *pro*A2 *rps*L20(Str ^{R}) *xyl*-5 λ*leumtl-*1)*,* Invitrogen^{™}

The host cells were cultured under conditions at 37 °C and 200 rpm using LB (Luria-Bertani) solid medium (Difco Laboratories, USA), and 100 µg/ml kanamycin or ampicillin was added to all mediums to prepare the host cells. Thereafter, a single colony of each cultured host cell was inoculated into LB liquid medium (Difco Laboratories, USA) for pre-culture, and then inoculated at 1% into SOB (Super Optimal Broth) medium and cultured at 18 °C for 24 hours, and then washed with Inoue TB buffer for preparation.

### (3) Production of transformed cells for mass production of vector

Each of the vectors prepared above was mixed with the host cells, and then the mixture was left on ice for 10 minutes and treated at 42 °C for 90 seconds and on ice for 3 minutes to produce transformed cells into which each vector was introduced. The produced transformed cells were plated on the LB solid medium containing antibiotics and then cultured at 37 °C. Through this, each vector was prepared in large quantities.

### 2. Production of recombinant lentiviral vector expressing E1 monobody-chimeric antigen receptor

A lentiviral vector was produced excluding the scFv sequence from the two lentiviral vectors prepared above (pLenti7.3::CD8α leader sequence-MSLN scFv-CD8 H&T-CD28-CD3ζ vector and pLenti7.3:: CD8α leader sequence-MSLN scFv-CD8 H&T-4-1BB-CD3ζ vector). First, inverse PCR was performed using two types of vectors as templates, using the CPL-F primer, CPL-R primer, and CloneAmp HiFi PCR premix (Takara) shown in Table 3 below. The amplified vector was isolated and purified to prepare two types of MSLN scFv-free chimeric antigen receptor vectors.

The E1 monobody sequence was prepared by amplifying the pETh-E1GSE1 vector by PCR using the E1-F primer and E1-R primer in Table 3 below, followed by isolation and purification.

**[Table 3]**

| SEQ ID NO | Sequence name | Sequence | Description |
|---|---|---|---|
| SEQ ID NO: 13 | CPL F | ACCACCACACCAGCTC C | plasmid linearization and ScFv sequence removal |
| SEQ ID NO: 14 | CPL R | AGGTCTAGCAGCATGC AG | plasmid linearization and ScFv sequence removal |
| SEQ ID NO: 15 | E1 F | CATGCTGCTAGACCTG TTTCTGATGTTCCGAG GGAC | E1 monomer, E1 dimer amplification |
| SEQ ID NO: 16 | E1 R | AGCTGGTGTGGTGGTT GTTCGGTAATTAATGG AAATTGGGC | E1 monomer, E1 dimer amplification |

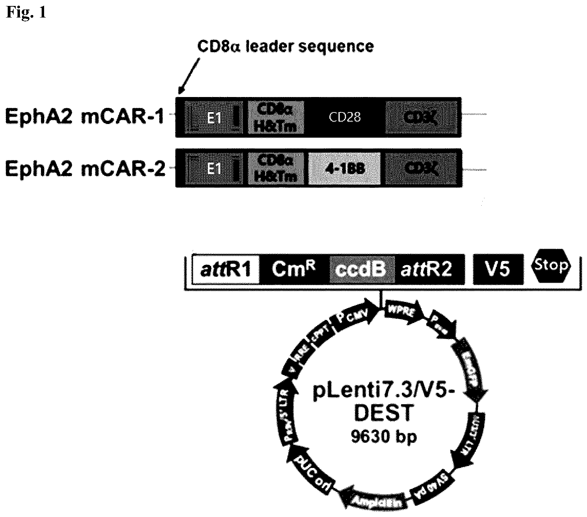

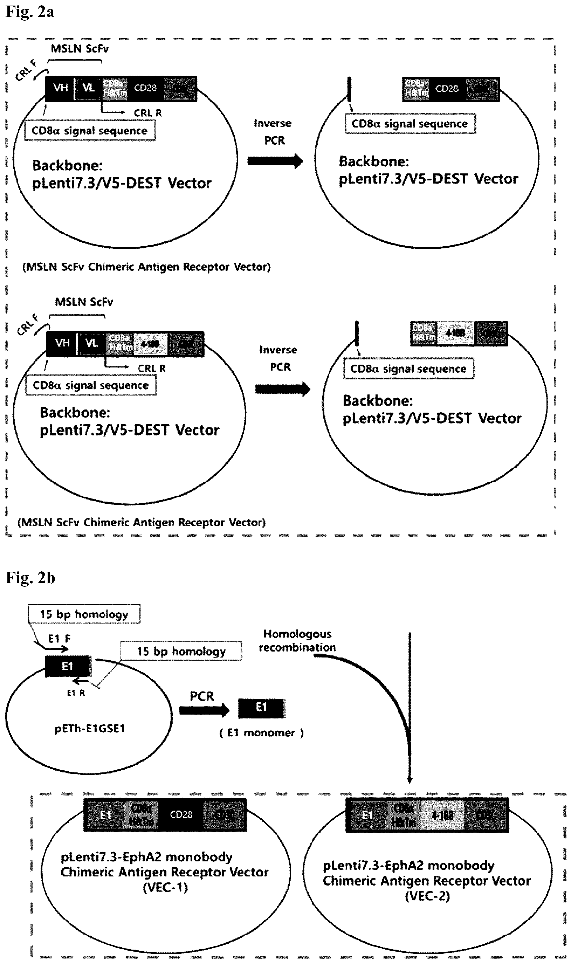

The prepared vector and E1 monobody sequence were inserted into the site where the scFv sequence was present through homologous recombination method using Overlap cloner DNA cloning kit (Elpisbio), thereby producing a recombinant lentiviral vector (pLenti7.3-EphA2 monobody chimeric antigen receptor vector) expressing a chimeric antigen receptor comprising an E1 monobody. The produced recombinant lentiviral vectors were designated as EphA2 mCAR-1 and EphA2 mCAR-2, respectively, and their structures are shown in Figure 1, and schematic diagrams of the vector production method are shown in Figures 2a and 2b.

### [Example 2]

### Production and evaluation of immune cells expressing E1 monobody-chimeric antigen receptor on cell membrane surface (VEC-1 and VEC-2)

### 1. Production of lentivirus comprising recombinant lentiviral vector

Lentiviruses comprising EphA2 mCAR-1 and EphA2 mCAR-2 prepared in Example 1 were prepared.

### 2. Preparation of immune cells and transduction via virus infection

### (1) Isolation of CD3+ T cells from PBMC

Peripheral blood mononuclear cells (PBMC) were isolated from human donor blood using Lymphoprep^{™} (STEMCELL). Thereafter, human CD3+ T cells were isolated from the isolated PBMCs by a positive selection method using CD3 microbeads (Miltenyi Biotech).

### (2) Activation of CD3+ T cells

Anti-CD3/anti-CD28 magnetic beads (anti-CD3/CD28 Dynabead; Gibco) and the isolated human CD3+ T cells were mixed at a ratio of 1:1. After 24 hours of mixing, the activated magnetic beads were removed using a MACSiMAG separator device. Thereafter, the cells were washed using RPMI-1640.

### (3) Transduction into lentivirus

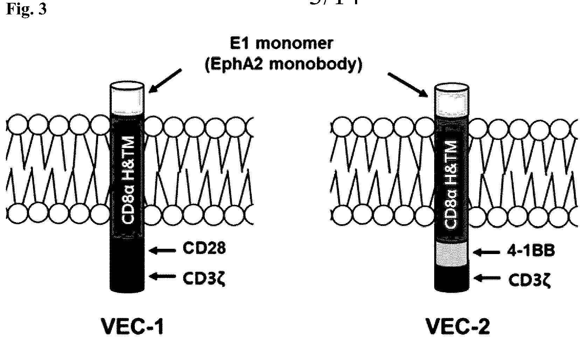

Lentivirus-infected T cells were produced by a virus infection method using polybrene (Sigma Aldrich). Specifically, the activated human CD3+ T cells were infected with lentiviruses comprising EphA2 mCAR-1 and EphA2 mCAR-2, respectively, and after 24 hours, the virus medium was replaced. Cell proliferation begins 48 hours after infection. The engineered immune cells thus prepared were designated as VEC-1 and VEC-2, respectively (including EphA2 mCAR-1 and EphA2 mCAR-2, respectively). The structure of the monobody-based chimeric antigen receptor expressed on the cell membrane surface is shown in Figure 3.

### 3. Evaluation of engineered immune cells

Whether the EphA2 mCAR-1 and EphA2 mCAR-2 vectors introduced into the VEC-1 and VEC-2 immune cells prepared above are properly expressed, and the activation status of these immune cells, were determined using FACS (fluorescence-activated cell sorting).

The Attune NxT flow cytometer (Thermo Fisher Scientific) was used in the experiment. Expression of the introduced vector was confirmed for each immune cell using an anti-GFP (Green Fluorescent Protein) antibody. Since the GFP expression site is present in the vector, the analysis results using an anti-GFP antibody will be high in immune cells that express this vector in large quantities. In addition, activation of T cells was confirmed using anti-CD25-PE antibody (PE Mouse Anti-Human CD25, BD Pharmingen^{™}) and anti-CD69-APC antibody (APC Mouse Anti-Human CD69, BD Pharmingen^{™}).

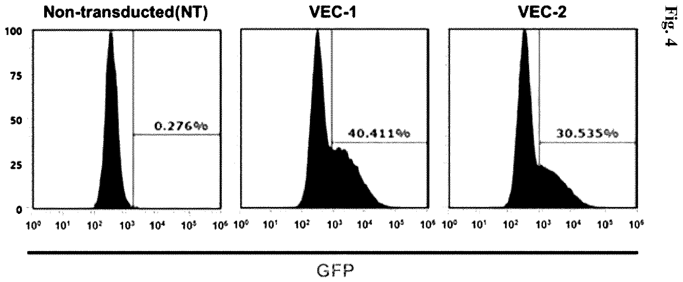

As a result of comparative analysis of the expression rate of the introduced vector (i.e., E1 monobody-chimeric antigen receptor sequence) by FACS, VEC-1 immune cells showed an expression rate of about 40% compared to the control group into which the vector was not introduced, and VEC-2 immune cells showed an expression rate of about 30% (see Figure 4). From these results, it was confirmed that the vector introduced into the immune cells was well expressed, and that the E1 monobody-chimeric antigen receptor was properly expressed within the engineered immune cells VEC-1 and VEC-2.

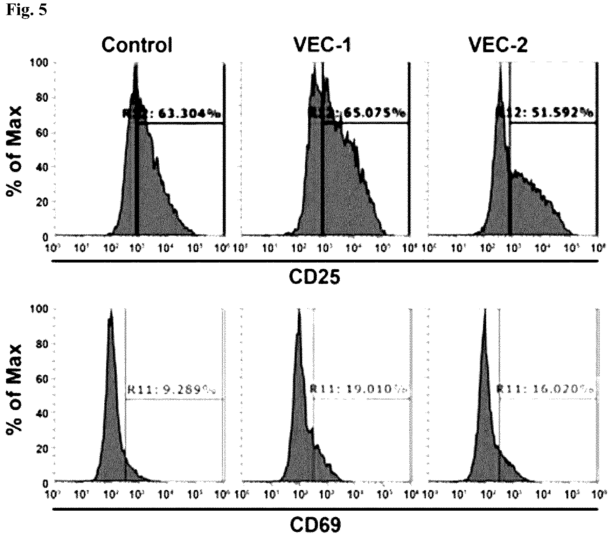

In addition, it was confirmed that in the engineered immune cells VEC-1 and VEC-2, CD25, a cell growth activity marker, was expressed similarly to the control group, and that CD69, a cytotoxic T cell activity marker, was expressed in greater amounts compared to the control group (see Figure 5). Therefore, it was confirmed that both VEC-1 and VEC-2 were active.

### [Example 3]

### Evaluation of cancer cell killing ability in vitro

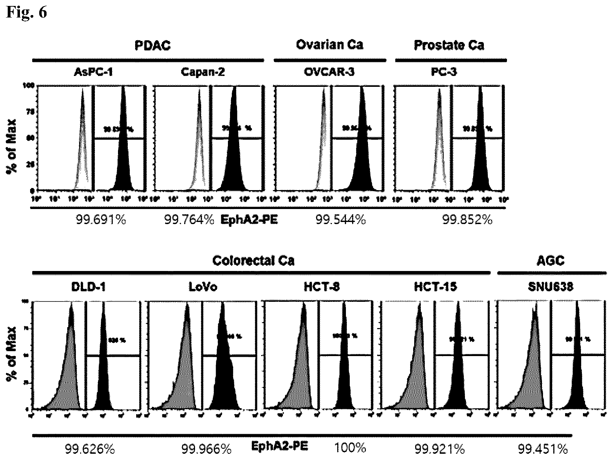

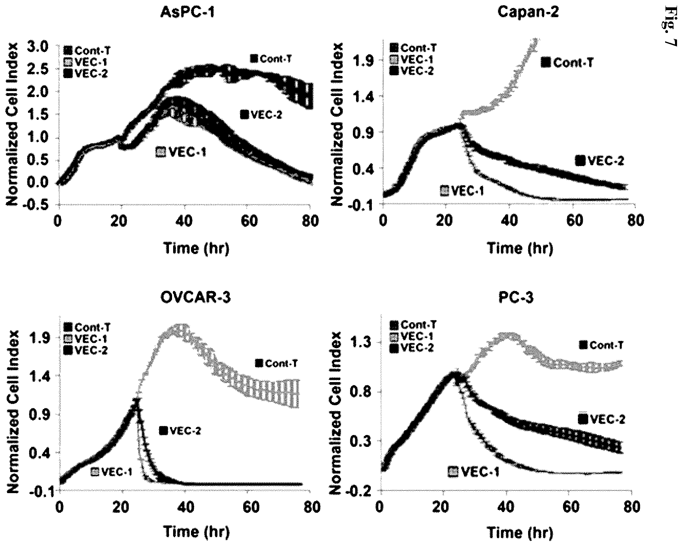

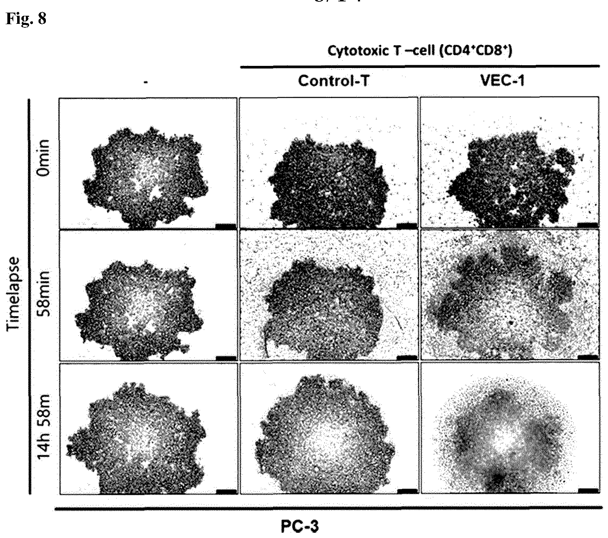

In order to evaluate the anticancer ability of the engineered immune cells VEC-1 and VEC-2 expressing the E1 monobody-chimeric antigen receptor against EphA2-specific cancer antigens, the cancer cells expressing EphA2 (ovarian cancer cell line OVCAR-3; pancreatic cancer cell line CAPAN-2 and AsPC-1; prostate cancer cell line PC-3, MSI-H stomach cancer cell line SNU638 and MSI-H large intestine cancer cell line DLD1, Lovo, HCT8, HCT15, see Figure 6) and the E1 monobody CAR-T cells (i.e., VEC-1 and VEC-2 cells) were co-cultured, and the cancer cell killing ability of the engineered immune cells was measured (see Figures 7 and 8).
(1) Ovarian cancer cell line OVCAR-3; pancreatic cancer cell lines Capan-2 and AsPC-1; and prostate cancer cell line PC-3; stomach cancer cell line SNU638, large intestine cancer cell lines DLD1, LoVo, HCT-8, and HCT-15 were purchased and used from the Korean Cell Line Bank.
   In order to confirm whether EphA2 was expressed in these cell lines, the expression and level of EphA2 antigen were analyzed by FACS using an anti-EphA2 antibody. As a result, it was confirmed that these cancer cell lines all express EphA2 at a high level (see Figure 6). Using this, the cancer cell killing ability of the E1 monobody CAR-T cells targeting EphA2 can be confirmed.
(2) The cancer cell lines in which expression of the EphA2 antigen was confirmed above were seeded in a 96 well plate at 1×10⁴ cells per well and placed in BSC for 30 minutes, and then the cancer cells were cultured in a cell incubator at 37 °C and 5% CO₂ conditions for 24 hours. Thereafter, the cancer cells were treated with 4×10⁴ E1 monobody CAR-T cells (i.e., VEC-1 and VEC-2 cells) per well and co-cultured for 72 to 90 hours, and the survival and cell movement activity of the cells were analyzed by RTCA as follows.
(3) Kinetic analysis by RTCA (xCELLigence, ACEA Biosciences, Inc.)
   Gold microelectrodes attached to the bottom surface of a microtiter plate (E-Plate) well were used as biosensors. When the microelectrodes are immersed in an electrically conductive solution (e.g. buffer or standard tissue culture medium) and a potential is applied across these electrodes (both terminals of the biosensor), electrons leave the cathode terminal. These electrons pass through the bulk solution and are deposited on the anode terminal, completing the circuit. The presence of adherent cells at the electrode-solution interface disrupts electron flow, because the signal varies depending on the interaction between the bulk solution and the biosensor. Therefore, by measuring the resistance value called impedance that occurs while cells grow, various cell responses (changes in cell number, proliferation rate, cell size, cell shape, and substrate attachment quality) can be automatically measured kinetically. The resistance value detected by the cell is displayed as an indicator called Cell Index (CI), and changes in the CI value show changes in the shape of the cell.
(4) As a result of comparative analysis of the killing ability of transduced E1 monobody CAR-T cells (VEC-1 or VEC-2) and non-transduced human T cells (control group) against cancer cells, it was confirmed by RTCA analysis that the killing ability of VEC-1 and VEC-2 was significantly increased (20-100%) compared to the control group (see Figure 7). From these results, it can be seen that the engineered immune cells according to the present invention have excellent cancer cell killing ability.
(5) The cancer cell killing ability of the E1 monobody CAR-T (VEC-1) immune cells using 3D co-culture was evaluated compared to the control group.

Prostate cancer cells (PC-3) were seeded in a 96 well plate at a cell number of 1 × 10³ cells. After culturing for 3 days, the sphere formed was co-cultured with VEC-1 at a cell number of 2 × 10³ in a medium. Thereafter, as a result of comparative analysis of the cancer cell killing ability of cytotoxic-T cell control group (Control-T) and VEC-1 using IncuCyte^{®} S3 Live-Cell Analysis System from Sartorius, it was observed that VEC-1 penetrates into the spherical structures of prostate cancer cell lines (PC-3), destroying the spherical structures and killing the cell lines, starting at 58 minutes after co-culture, earlier than the control group. At 14 hours and 58 minutes of co-culture, it was observed that VEC-1 completely destroyed the spherical structures of prostate cancer cells (PC-3), whereas the cytotoxic-T cell control group was observed in the center of the spherical shape while maintaining its spherical shape without destroying the shape of the spherical structure (see Figure 8).

### [Example 4]

### Evaluation of anti-tumor capacity of immune cells in xenograft mouse model

All animal experiments were conducted with approval from the Institutional Animal Care and Use Committee (IACUC, Chonnam National University, Republic of Korea). Four-week-old female NSG (NOD SCID gamma) mice were supplied from SPF animal laboratory of Vaccine Center at Hwasun.

Pancreatic cancer cells AsPC-1/luc were suspended in a 1:1 mixed solution of PBS (Welgene) and high-concentration Matrigel (Corning) at a concentration of 4 × 10⁶ cells/200 ul to 8 × 10⁶ cells/200 ul. PC-3 cells were suspended in a 1:1 mixed solution of PBS (Welgene) and high-concentration Matrigel (Corning) at a concentration of 3 × 10⁶ cells/100 ul to 1 × 10⁷ cells/100 ul. In the experiment, AsPC-1/luc (2 × 10⁶ cells) and PC-3(3 × 10⁶ cells) were injected subcutaneously into the right flank of the mouse.

When the tumor size reached 100 to 150 mm³, the prepared PBS, control group T cells, VEC-1 and VEC-2 immune cells were injected intravenously once at 1 × 10⁷/200 ul. Five to ten mice were used per group, and the tumor size and body weight of the mice were measured twice a week.

### 1. Cell thawing and cell culture

**Experimental materials:** RPMI 1640 (Gibco), FBS (Gibco), P/S (Gibco), L-glutamine 200 mM (Gibco), 100∲ cell culture flask, basket, tumor cell lines (AsPC-1 and PC-3), Solution 18 AO DAPI (ChemoMetec), PBS (Welgene), TrypLE^{™} (Gibco). Human pancreatic cancer cell line AsPC-1 and human prostate cancer cell line PC-3 were supplied from ATCC (American Type Culture Collection). The human pancreatic cancer cell line AsPC-1/luc, which expresses luciferase, was supplied from JCRB (Cell bank, Austrailia).

**Cell thawing:** In the liquid nitrogen tank, the tumor cell line was placed in a 37 °C constant temperature water bath and quickly thawed. Thereafter, when the thin ice was floating, it was transferred to a clean bench and placed in a 15 mL conical tube. 9 mL of cell culture medium (RPMI 1640 + 10 % FBS + 1% P/S + 2 mM L-glutamine) was placed in a 15 ml tube and centrifuged at 350g for 3 minutes. The supernatant was removed, and 1 mL of cell culture solution was added and completely resuspended, and then 9 mL of cell culture solution was added to make 10 mL and resuspended. The number of cells was calculated using a cell count solution (PBS: Solution 18 = 90 : 5), and the cells were spread in a 100∲ flask so that the cell density was 0.5 × 10⁶/10 mL and 1 × 10⁶/10 mL (for PC-3 and AsPC-1, respectively). Thereafter, subculture was performed at 3-day intervals (when the cell density reached 70-80%).

**Subculture:** The supernatant was removed from the flask and then washed by adding 10 mL of PBS to remove impurities such as cell debris, and then the supernatant was discarded. In order to detach the cells from the flask, TrypLE^{™} (2 mL or 4 mL) was added to the 100∲ and 150∲ flasks, respectively. When the cells were floating in a round shape upon observing under a microscope, 10 mL or 30 mL of cell culture medium (RPMI 1640 + 10 % FBS + 1% P/S + 2 mM L-glutamine) was added, and the cells were placed in a 50 ml conical tube and centrifuged at 500 g for 3 to 5 minutes. The supernatant was removed and then resuspended by adding 1 mL of cell culture solution, and then 9 mL of cell culture solution was added. The number of cells was measured using a cell count solution (PBS : Solution 18 = 90 : 5), and then the cancer cells (e.g., pancreatic cancer cells AsPC-1: 1×10⁶/10 mL) were placed in a culture vessel and cultured.

### 2. Preparation of mouse

**Experimental materials:** PICO 5053 (Orient Bio), g-irradiated 18 % beta-chip (Orient Bio), sterilized tap water and water bottle, high-pressure steam sterilizer, clipper (Jeung Do Bio & Plant Co., Ltd.), wet tissues, Ifran solution (Hana Pharm Co., Ltd.), depilatory agent (veet), respiratory anesthesia machine and chamber

**Experimental method:** Feed (PICO 5053), litter (r-irradiated 18 % beta-chip), sterilized tap water, and cage were replaced twice a week. Hair was removed 3 days before the tumor was implanted, and if the hair grew enough to make the tumor difficult to see during the experiment, the hair was removed. The mouse was anesthetized by breathing with isoflurane at a concentration of 2-3%, and the hair on the right side of the mouse was removed with a clipper. A depilatory agent was applied and left on for about 1 minute, and the depilatory agent was wiped off with a wet tissue.

### 3. Preparation of human tumor xenograft mouse model

**Experimental materials:** cells, 1 ml syringe (Korea Vaccine Co., Ltd.), 25 ½ G needle (Korea Vaccine Co., Ltd.), high-concentration Matrigel (Corning), ice box, EP tube, respiratory anesthesia machine and chamber, Ifran solution (Hana Pharm Co., Ltd.). In the case of Matrigel, it was placed in the refrigerator overnight the day before xenograft. Because Matrigel gels at room temperature, the experiment was always performed on ice.

**Experimental method:** The mouse was anesthetized by breathing with isoflurane at a concentration of 2-3%, and then 100 ul of a 1:1 mixed solution of AsPC-1/luc (2 × 10⁶ cells) and PC-3 (3 × 10⁶ cells) with Matrigel was injected subcutaneously into the right flank of the mouse. In addition, 100 ul of a 1:1 mixed solution of tumor cell lines (4 × 10⁶ cells/100 ul of PBS) and matrigel was injected subcutaneously into the abdominal cavity of each mouse.

### 4. Injection of chimeric antigen receptor T cells

**Experimental materials:** 27 ½ G needle (Jeonglim), 1 ml syringe (Korea Vaccine Co., Ltd.), PBS, normal T cells (control group), E1 monobody CAR-T cells (VEC-1 and VEC-2), mouse calibrator (Jeung Do Bio & Plant Co., Ltd.), heat irradiator (Jeung Do Bio & Plant Co., Ltd.), 70 % alcohol swab, vernier caliper (Jeung Do Bio & Plant Co., Ltd.), scale, camera.

**Experimental method:** The tumor size and body weight of the mice were measured, and the tumor size and body weight of the mice were adjusted to be similar for each group. When the tumor size reached 100 to 150 mm³, 200 ul of PBS and the cells (1 × 10⁷/200 ul of control normal T cells and E1 monobody CAR-T cells) were injected intravenously into the tail vein of the mouse.

### 5. Mouse monitoring (measurement of tumor size and body weight)

**Experimental materials:** respiratory anesthesia machine and chamber, Ifran solution (Hana Pharm Co., Ltd.), vernier caliper (Jeung Do Bio & Plant Co., Ltd.), scale, camera.

**Experimental method:** The mouse was anesthetized by breathing with isoflurane at a concentration of 2-3%, and then the tumor size and body weight were measured twice a week. The tumor size of the mouse was calculated by measuring the long axis (L), short axis (W), and height (H) with a vernier caliper and then multiplying by 0.52, and the body weight of the mouse was measured using an electronic scale.

### 6. Experimental results

### (1) Experiment using prostate cancer cell line

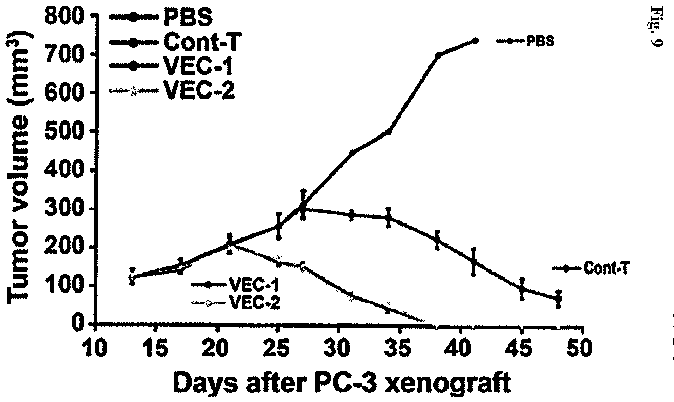

Twelve days after tumor formation in a mouse model in which a prostate cancer cell line (PC-3) at 1 × 10⁷ cells/100 ul and 1 × PBS were xenografted into the subcutaneous tissue of the right back of the mouse, each group was injected intravenously with PBS, control group T cells (activated control normal T cells that were not transduced with virus; Cont-T), VEC-1 cells, and VEC-2 cells, and then the tumor size was measured using a scale bar, and the results are shown in Figure 9.

As shown in Figure 9, from the 22nd day after tumor formation, the size of the tumor was significantly reduced in mice injected with VEC-1 or VEC-2 compared to mice injected with control normal T cells or only PBS.

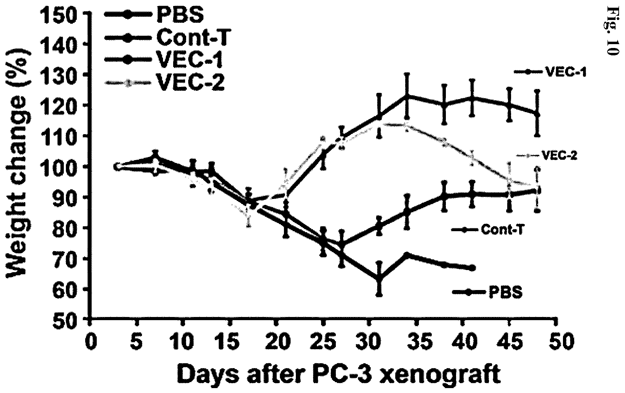

In order to determine whether the mouse's health condition recovered after intravenous administration of E1 monobody CAR-T cells and whether there were side effects due to administration, the body weight of the mouse was measured, and the results are shown in Figure 10.

As shown in Figure 10, the mice administered with VEC-1 or VEC-2 recovered their health condition more quickly than the mice administered with control normal T cells, and then the mice administered with normal T cells showed recovery after 30 days. The mice administered with only PBS continued to lose body weight in proportion to the tumor size, and their health condition deteriorated.

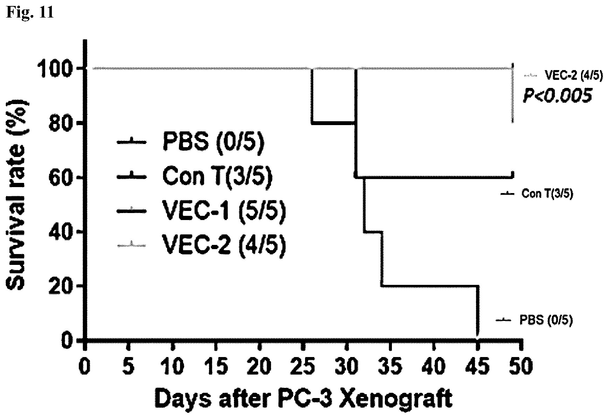

The survival rate of the prostate cancer xenograft mouse models at the end of the experiment (day 50) was evaluated using the Kaplan-Meier formula, and the results are shown in Figure 11. As shown in Figure 11, the mouse groups administered with VEC-1 and VEC-2 both showed high survival rates, which were significantly superior to the mouse group administered with the control normal T cells.

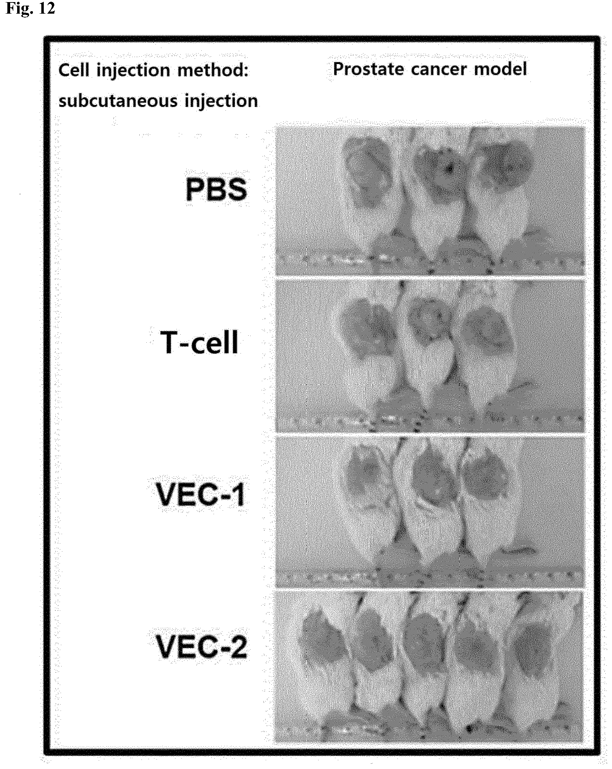

On the 44th day after implanting the tumor on the right back of the mouse, E1 monobody CAR-T cells (VEC-1 or VEC-2) were injected intravenously. The size of the tumor was measured on the 28th day after injection, and the results are shown in Figure 12. As shown in Figure 12, it was confirmed that the tumors in the mouse group administered with VEC-1 or VEC-2 were significantly smaller than those in the mouse group administered with normal T cells and the mouse group administered with only PBS.

### (2) Experiment using pancreatic cancer cell line

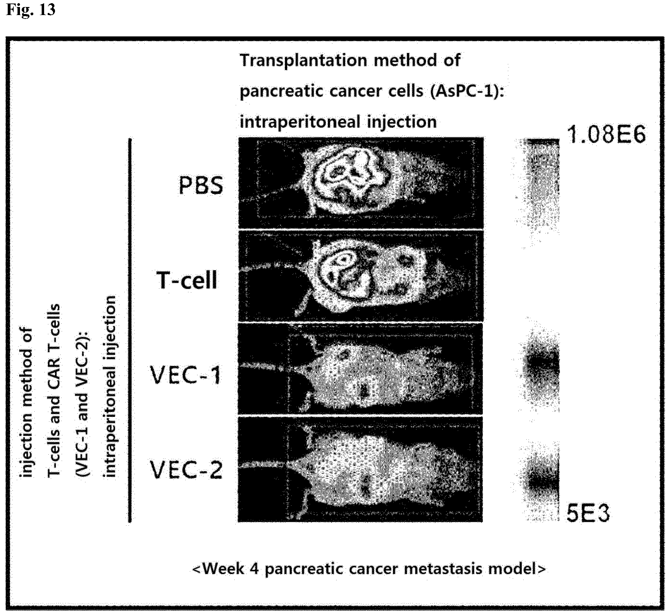

Pancreatic cancer cells (AsPC-1, 2 × 10⁶ cells) were transplanted into the mouse abdominal cavity. After 14 days, PBS, normal T cells, VEC-1, and VEC-2 were administered intraperitoneally. At 4 weeks after administration, the degree of metastasis of pancreatic cancer cells was imaged using an In Vivo Imaging System (IVIS), and the results are shown in Figure 13. As shown in Figure 13, it was confirmed that tumors were hardly proliferated or metastasized in the mice administered with VEC-1 and VEC-2 compared to the mice administered with only normal T cells and PBS.

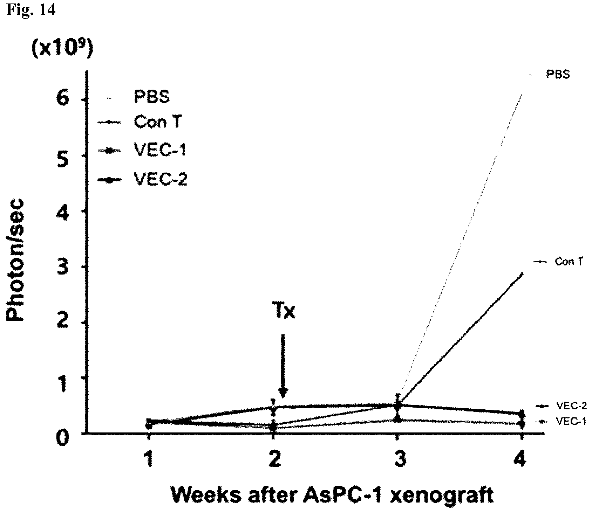

2 × 10⁶ pancreatic cancer cells were transplanted into the abdominal cavity of the mouse. 14 days after transplantation, PBS, normal T cells (Con T), VEC-1, and VEC-2 were administered intraperitoneally, and the size of the tumor was measured using Bioluminescence Imaging (BLI). The results are shown in Figure 14. As shown in Figure 14, the tumors were rarely proliferated in the mice administered with VEC-1 or VEC-2 compared to the mice administered with normal T cells and the mice administered with only PBS, which persisted until 4 weeks after transplantation of pancreatic cancer cells.

It can be seen that the monobody-based chimeric antigen receptor according to the present invention and the immune cells (e.g., cytotoxic T cells) expressing the same on the cell membrane surface have excellent anticancer effects that significantly inhibit the proliferation of prostate cancer and pancreatic cancer cells and significantly increase the survival rate of mice. Therefore, the immune cells comprising the monobody-based chimeric antigen receptor can be used to prevent or treat various cancers, including solid cancers.

## Claims

1. A monobody-based chimeric antigen receptor comprising an extracellular ligand binding domain comprising a monobody.

2. The monobody-based chimeric antigen receptor according to claim 1, wherein the monobody specifically binds to a protein selected from the group consisting of CRL1, ephrin receptor, EphA2, β-galactosidase, RAS, Abl kinase, VEGFR2, MBP, SARS-CoV-2, Bcr-Abl kinase, STAT3, EGFR, VEGFR2, SH3 domain of human Lyn tyrosine kinase, MLKL, Fluc homologues, mitogen-activated protein kinase (MAPK), ERK2, MAPK14, kinase SH2 domain, SUMO, AurA, WDR5, Flue family, GFP, receptor binding domain of SARS-CoV-2, SH3 domain of FYN, SH2 domain of ABL, SUMO1, Bcr-Abl, GPR56 ECR, PD-L1, glypican-3, PCSK9, Gp41, CD4, and IL-23.

3. The monobody-based chimeric antigen receptor according to claim 2, wherein the monobody specifically binds to ephrin receptor, EphA2, or human EphA2.

4. The monobody-based chimeric antigen receptor according to claim 3, wherein the monobody comprises the amino acid sequence of SEQ ID NO: 8.

5. The monobody-based chimeric antigen receptor according to claim 1, wherein the monobody-based chimeric antigen receptor further comprises at least one selected from the group consisting of a transmembrane domain; and an intracellular signaling domain.

6. The monobody-based chimeric antigen receptor according to claim 5, wherein the transmembrane domain is at least one selected from the group consisting of T-cell receptor alpha chain, T-cell receptor beta chain, T-cell receptor zeta chain, CD8 alpha chain, CD8 beta chain, CD28, CD3epsilon, CD45, CD4, CD5, CD8, CD9, CD16, CD22, CD33, CD37, CD64, CD80, CD86, CD134, CD137, CD154, a portion thereof, and a combination thereof.

7. The monobody-based chimeric antigen receptor according to claim 5, wherein the intracellular signaling domain is at least one selected from the group consisting of TCRzeta, FcRgamma, FcRbeta, FcRepsilon, CD3gamma, CD3delta, CD3epsilon, CD3zeta, CD5, CD22, CD79a, CD79b, and CD66d.

8. The monobody-based chimeric antigen receptor according to claim 5, wherein the intracellular signaling domain is at least one selected from the group consisting of an OX40 domain, a CD2 domain, a CD27 domain, a CD28 domain, a CDS domain, an ICAM-1 domain, a LFA-1 domain, and a 4-1BB domain.

9. The monobody-based chimeric antigen receptor according to claim 5, wherein the monobody-based chimeric antigen receptor further comprises a hinge.

10. A polynucleotide comprising a nucleic acid sequence encoding the monobody-based chimeric antigen receptor according to any one of claims 1 to 9.

11. An expression vector comprising the polynucleotide according to claim 10.

12. An immune cell expressing the monobody-based chimeric antigen receptor according to any one of claims 1 to 9 on a cell surface membrane.

13. The immune cell according to claim 12, wherein the immune cell is a white blood cell, a neutrophil, an eosinophil, a basophil, a monocyte, a lymphocyte, a T cell, a cytotoxic T cell, a natural killer T cell, a dendritic cell, or a combination thereof.

14. A pharmaceutical composition for preventing or treating cancer, comprising the immune cell according to claim 12.

15. The pharmaceutical composition for preventing or treating cancer according to claim 14, wherein the cancer is at least one selected from the group consisting of melanoma, squamous cell carcinoma, breast cancer, head and neck cancer, thyroid cancer, soft tissue sarcoma, osteosarcoma, testicular cancer, prostate cancer, ovarian cancer, bladder cancer, skin cancer, brain cancer, angiosarcoma, mast cell tumor, leukemia, lymphoma, liver cancer, lung cancer, pancreatic cancer, stomach cancer, kidney cancer, large intestine cancer, hematopoietic tumor, and a metastatic cancer thereof.

16. The pharmaceutical composition for preventing or treating cancer according to claim 15, wherein the cancer is pancreatic cancer, prostate cancer, or ovarian cancer.
